# EUROPEAN PATENT SPECIFICATION

(11) **EP 2 026 651 B1**
(45) Date of publication and mention of the grant of the patent: **17.04.2013**
(21) Application number: 07752686.1
(22) Date of filing: 08.03.2007
(51) Int. Cl.: A61K 31/455, A61K 31/60, A61K 45/06

(54) **COMBINATION THERAPY WITH NON-SELECTIVE COX INHIBITORS TO PREVENT COX-RELATED GASTRIC INJURIES**
KOMBINATIONSTHERAPIE MIT NICHTSELEKTIVEN COX-INHIBITOREN ZUR VERHINDERUNG MIT COX VERBUNDENER MAGENVERLETZUNGEN
POLYTHÉRAPIE PRÉSENTANT DES INHIBITEURS COX NON SÉLECTIFS EN VUE DE PRÉVENIR DES LÉSIONS GASTRIQUES LIÉES À COX

(30) Priority: 08.03.2006 US 780264 P
(43) Date of publication of application: 25.02.2009
(73) Proprietor: Cortria Corporation, Needham, MA 02494 (US)
(72) Inventor: KRANTZ, Alexander, Boston, MA 02118 (US)
(74) Representative: Sitkowska, Jadwiga
(86) International application number: PCT/US2007/006004
(87) International publication number: WO 2007/103540

(56) References cited:
- EP-A- 0 420 368
- WO-A-00/40559
- WO-A1-00/40559
- WO-A2-2005/067927
- US-A- 4 123 441
- US-A- 5 093 325
- US-A- 6 022 884
- US-B1- 6 342 524
- US-B1- 6 380 218
- US-B1- 6 740 340
- TASCILAR O; SARAY A; DIZBAY-SAK S; MELLI M: "Acetylsalicylic acid and misoprostol combination in adjuvant arthritis of rats" INFLAMMATION, vol. 17, no. 4, 1993, pages 489-498, XP009129080
- CHIU L C M; TONG K F; OOI V E C: "Cytostatic and cytotoxic effects of cyclooxygenase inhibitors and their synergy with docosahexaenoic acid on the growth of human skin melanoma A-375 cells" BIOMEDICINE & PHARMACOTHERAPY, vol. 59, no. Suppl.2, October 2005 (2005-10), pages S293-S297, XP002567155
- SUEHIRO A ET AL: "COMBINATION EFFECT OF EICOSAPENTAENOIC ACID AND PLATELET SUPPRESSIVE AGENTS ON PLATELETS" CURRENT THERAPEUTIC RESEARCH, EXCERPTA MEDICA, TRENTON, NJ, US, vol. 55, no. 6, 1 June 1994 (1994-06-01), pages 653-659, XP009013937 ISSN: 0011-393X
- MURAKAMI AKIRA; TAKAHASHI DAISUKE; HAGIHARA KAZUMA; KOSHIMIZU KOICHI; OHIGASHI HAJIME: "Combinatorial effects of nonsteroidal anti-inflammatory drugs and food constituents on production of prostaglandin E2 and tumor necrosis factor-alpha in RAW264.7 murine macrophages" BIOSCIENCE BIOTECHNOLOGY AND BIOCHEMISTRY, vol. 67, no. 5, May 2003 (2003-05), pages 1056-1062, XP002567156
- ISDALE AMANDA; WRIGHT VERNA: "Misoprostol/NSAID fixed combinations: Help or hindrance in clinical practice?" DRUG SAFETY, vol. 12, no. 5, 1995, pages 291-298, XP009129093

## Description

Non-steroidal anti-inflammatory drugs (NSAIDs), including compounds such as ibuprofen, ketoprofen and diclofenac, have anti-inflammatory actions and are effective in pain associated with the release of prostaglandins and other mediators of inflammation. For example, diclofenac is considered to be extremely potent and effective as an analgesic and anti-inflammatory agent. Diclofenac is approved in the United States for the long-term symptomatic treatment of rheumatoid arthritis, osteoarthritis and alkylosing spondylitis. It is also considered to be useful for the short-term treatment of acute musculoskeletal injury, acute painful shoulder, post-operative pain and dysmenorrhea. Furthermore, NSAIDs have been widely used in arthritis therapy for several years.

Although NSAIDs are widely accepted as effective agents for controlling pain, their administration can lead to the development of gastroduodenal lesions, e.g., ulcers and erosions, in susceptible individuals. It has been suggested that a major factor contributing to the development of these lesions is the presence of acid in the stomach and upper small intestine of patients. This view is supported by clinical studies demonstrating an improvement in NSAID tolerability when patients are also taking independent doses of acid inhibitors (Dig. Dis. 12:210-222 (1994); Drug Safety 21: 503- 512 (1999); Aliment. Pharmacol. Ther. 12: 135-140 (1998); Am. J. Med. 104(3A):67S- 74S (1998); Clin. Ther. 17: 1159-1 173 (1995)). Other major factors contributing to NSAID-associated gastropathy include a local toxic effect of NSAIDs and inhibition of protective prostaglandins (Can. J. Gastroenterol. 13: 135-142 (1999) and Pract. Drug Safety 21 :503-512, (1999)), which may also make some patients more susceptible to the ulcerogenic effects of other noxious stimuli.

Attempts to develop NSAIDs that are inherently less toxic to the gastrointestinal tract have met with only limited success. For example, the recently developed cyclooxygenase-2 (COX-2) inhibitors show a reduced tendency to produce gastrointestinal ulcers and erosions, but a significant risk is still present, especially if the patient is exposed to other ulcerogens (JAMA 284: 1247-1255 (2000); N. Eng. J. Med. 343: 1520-1528 (2000)). In addition, the COX-2 inhibitors may not be as effective as other NSAIDs at relieving some types of pain and have been associated with significant cardiovascular problems (JADA 131 : 1729-1737 (2000); SCRIP 2617, pg. 19, Feb. 14, 2001).

Other attempts to produce an NSAID therapy with less gastrointestinal toxicity have involved the concomitant administration of a cytoprotective agent. In 1998, Searle began marketing Arthrotec™ for the treatment of arthritis in patients at risk for developing GI ulcers. This product contains misoprostol (a cytoprotective prostaglandin) and the NSAID diclofenac. Although patients administered Arthrotec™ do have a lower risk of developing ulcers, they may experience a number of other serious side effects such as diarrhea, severe cramping and, in the case of pregnant women, potential damage to the fetus. Another approach has been to produce enteric coated NSAID products.

However, even though these have shown modest reductions in gastroduodenal damage in short term studies (Scand. J. Gastroenterol. 20: 239-242 (1985) and Scand. J. Gastroenterol. 25:231-234 (1990)), there is no consistent evidence of a long term benefit during chronic treatment.

Overall, it may be concluded that the risk of gastrointestinal toxicity in the form of gastritis, peptic erosions, ulcerations, GI bleeds, etc., is a recognized problem associated with the administration of NSAIDs and that, despite considerable effort, an ideal solution has not yet been found.

Tascilar O; Saray A; Dizbay-Sak S; Melli M. in Inflammation, Vol. 17, No. 4, 1993, pages 489-498 report that misoprostol increases the inflammatory effect of acetylsalicylic acid in a rat model of rheumatoid arthritis.

WO00/40550 discloses pharmaceutical composition comprising 1-methylnicotinamide salt for treating skin diseases and mentions that said compound can be formulated for topical application in dermatological disorders and cosmetic application combined with 0,5-10% salicylic acid.

US-B1-6342524 teaches a composition comprising a prostaglandin F agonist, like latanoprost, and a NSAID nepafenac and its derivatives.

Chiu L.C.M. et al. in Biomedicine Et Pharmacotherapy, Vol. 59, No. Suppl. 2, October 2005, pages S293-S297, mentions a composition comprising docosahexanoic acid and a NSAID (indomethacin, aspirin).

EP-A-0420368 reports a composition comprising acetylsalicylic acid and one of cicaprost/eptaloprost.

Suehiro A. et al. in Current Therapeutic Research Excerpta Medica, Vol. 56, No. 6, 1994, pages 653-659, disclose a composition comprising eicosahexanoic acid and acetylsalicylic acid.

Murakami A. et al., in Bioscience Biotechnology and Biochemistry, Vol. 67, No. 5, 2003, pages 1056-1062 report synergistic effect of the combinations aspirin + genistein and indomethacin + genistein on the formation of proinflammatory mediators.

Isdale A. et al., in Drug Safety, Vol. 12, No. 5, 1995, pages 291-298 report that NSAIDs cause toxicity to the gastrointestinal tract and suggests the co-administration of prostaglandins such as misoprostol to reduce NSAIDs induced gastrointestinal ulceration.

US-B1-6740340 teaches tablets comprising NSAID and misoprostol for counteracting ulcerogenic effects of NSAID administration.

US-A-4123441 discloses prostacyclin analogs and mentions their potential utility in reducing the undesirable gastrointestinal effects resulting from systemic administration of anti-inflammatory prostaglandin synthetase inhibitors, for example indomethacin and aspirin.

### Summary of the Invention

In one aspect, the invention provides a pharmaceutical composition formulated for oral administration comprising an NSAID and a prostaglandin mimetic. The prostacyclin mimetic is a 1-methylnicotinamide salt, and the composition is formulated for oral administration The 1-methylnicotinamide salt is represented by formula (I): 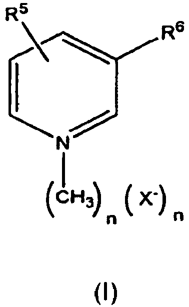 wherein R⁵ is H; R⁶ is -CONH₂; n is 1; and X⁻ is a physiologically suitable counter-anion.

In an embodiment, the invention provides the pharmaceutical composition of the invention, for use in the treatment or prevention of deleterious effects associated with NSAID administration in a subject. In one embodiment, the deleterious effects associated with NSAID administration in a subject are related to a decrease in one or more prostaglandins anywhere in the gastrointestinal tract. In another embodiment, the deleterious effect associated with NSAID administration in a subject is GI toxicity. In yet another embodiment, the GI toxicity is selected from the group consisting of gastritis, peptic erosions, ulceration, gastric lesions and GI bleeds.

In another embodiment, the NSAID for use in the invention is selected from the group consisting of aspirin, indomethacin, ibuprofen, naproxen, fenoprofen, tolmetin, sulindac, meclofenamate, ketoprofen, piroxicam, flurbiprofen, diclofenac, acetylsalicylic acid, sodium acetylsalicylate, calcium acetylsalicylate, salicylic acid, sodium salicylate, choline salicylate, magnesium salicylate, salsalate, sodium salicylate, diflunisal, ketorolac, carprofen, mefenamic acid, meloxicam and nimesulide.

In another aspect, the composition of the invention is used in a method of treating pain and/or inflammation in a subject, said method comprising administering to the subject in need thereof a therapeutically-effective amount of a pharmaceutical composition comprising an NSAID and a molecule that selectively stimulates the release of a prostacyclin, i.e. the compound of formula (I).

In another embodiment, the inflammation is selected from the group consisting of fever, arthritis, asthma, bronchitis, menstrual cramps, tendinitis, bursitis, inflammatory disorders of the skin, gastrointestinal conditions, vascular diseases, migraine headaches, periarteritis nodosa, thyroiditis, aplastic anemia, Hodgkin's disease, sclerodoma, rheumatic fever, myasthenia gravis, sarcoidosis, nephrotic syndrome, Behcet's syndrome, polymyositis, hypersensitivity, conjunctivitis, gingivitis, swelling occurring after injury and myocardial ischemia. In yet another embodiment, the arthritis is selected from rheumatoid arthritis, spondyloarthropathies, gouty arthritis, systemic lupus erythematosus, osteoarthritis and juvenile arthritis.

In still another embodiment, the inflammatory disorders of the skin are selected from the group consisting of psoriasis, eczema, burns and dermatitis.

In another embodiment, the gastrointestinal conditions are selected from the group consisting of inflammatory bowel syndrome, Crohn's disease, gastritis, irritable bowel syndrome and ulcerative colitis.

In yet another embodiment, the pain is selected from the group consisting of menstrual pain, low back pain, neck pain, skeletal pain, post-partum pain, headache, pain associated with migraine, toothache, sprains, strains, arthritis, degenerative joint diseases, gout, ankylosing spondylitis, bursitis, burns, including radiation and corrosive chemical injuries, sunburns, bone fracture, immune and autoimmune diseases, cellular neoplastic transformations or metastic tumor growth, and pain following surgical and dental procedures.

In another embodiment, the arthritis is selected from rheumatoid arthritis, spondyloarthopathies, gouty arthritis, systemic lupus erythematosus, osteoarthritis and juvenile arthritis.

In still another embodiment, the therapeutically-effective amount of a pharmaceutical composition comprising an NSAID and the compound of formula (I) is used to treat gastric lesions, symptoms of GI toxicity, ischemia, reperfusion, colorectal cancer and damage to the gastric mucosa in a subject. In yet another embodiment, the symptoms of GI toxicity are selected from the group consisting of gastritis, peptic erosions, ulcerations and GI bleeding.

In a preferred embodiment, X⁻ is chloride, benzoate, salicylate, acetate, citrate or lactate.

In yet another preferred embodiment, the compound of Formula I is selected from 1-methylnicotinamide chloride, 1-methylnicotinamide citrate, and 1-methylnicotinamide lactate.

### Detailed Description of the Invention

The present invention is directed to a pharmaceutical composition comprising one or more NSAID and a prostaglandin mimetic, i.e. the 1-methylnicotinamide salt, the composition being formulated for oral administration, and its use in treating diseases and disorders, such as those related to pain and inflammation. The prostaglandin mimetic selectively stimulates the release of PGI2. The prostaglandin mimetic serves to counteract the deleterious side effects associated with NSAID administration.

Complications arising from chronic NSAID use are common and are primarily due to gastrointestinal (GI) toxicity in the form of, for example, gastritis, peptic erosions, ulceration, gastric lesions and GI bleeds. This GI toxicity has been attributed to the blockade of the isoform cyclo-oxygenase (COX)-1 which is the primary isoform responsible for the production of cytoprotective prostaglandins (PGE2 and PGI2) in the stomach. However, this hypothesis appears to be oversimplified.

In a series of articles dealing with selective and unselective COX inhibitors (reviewed in "COX inhibition and NSAID-induced gastric damage-roles in various pathogenic events." Curr. Top. Med. Chem. 2005; 5(5):475-86) Takeuchi et al. have investigated the relation between COX inhibition and pathogenic events in rodents. Takeuchi et al. conclude that (1) the gastric ulcerogenic properties of NSAIDs are not accounted for solely by the inhibition of COX-1 and require the inhibition of both COX-1 and COX-2, (2) the inhibition of COX-1 up-regulates COX-2 expression in association with gastric hypermotility, and (3) prostaglandins produced by COX-2 counteract the deleterious influences of the COX-1 inhibition.

In light of these observations, a strategy designed to stimulate the production of one or more prostaglandins in a subject that are otherwise depleted by the administration of NSAIDs is warranted, and is the subject of this invention. Without being bound by theory, it is believed that the stimulation of these prostaglandins serves to counteract the contributions of NSAID-induced COX inhibition to injuries of the gastrointestinal tract. The production of such depleted prostaglandins can be amplified through the use of a prostaglandin mimetic. The prostaglandin mimetic is administered with one or more NSAIDs (e.g., naproxen, diclofenac, or indomethacin) to a subject as a pharmaceutical composition formulated for oral administration.

For example, the major prostaglandins produced by COX-2 are PGE2 and PGI2. As discussed above, NSABD-induced depletion of either of these prostaglandins may lead to GI toxicity. Therefore, replenishing the concentrations of either or both of these prostaglandins will counteract these adverse effects. MNA, also referred to as 1-methylnicotinamide, has been demonstrated in a thrombolytic assay to stimulate the secretion of PGI2 (WO05067927A3). Thus, MNA could be co-administered, in appropriate proportions, with one or more NSAIDs to counteract adverse effects in the GI tract by the enhanced production of PGI2. A pharmaceutical composition of this sort will also be effective for the treatment of NSAID-associated disease and disorders such as pain and inflammation.

Similarly, a pharmaceutical composition comprising one or more NSAIDs and a prostaglandin mimetic can be used for the treatment of NSAID-associated diseases and disorders such as pain and inflammation by oral administration.

### Definitions

These and other embodiments of the invention will be described with reference to following definitions that, for convenience, are collected here.

As used herein, the term "NSAID" includes, but is not limited to, those agents which inhibit cyclooxygenase, the enzyme responsible for the biosyntheses of the prostaglandins and certain autocoid inhibitors, including inhibitors of the various isoenzymes of cyclooxygenase (including, but not limited to, cyclooxygenase-1 and -2), such as the commercially available NSAIDs aceclofenac, acemetacin, acetaminophen, acetaminosalol, acetyl-salicylic acid, acetyl-salicylic-2-amino-4-picoline-acid, 5-aminoacetylsalicylic acid, alclofenac, aminoprofen, amfenac, ampyrone, ampiroxicam, anileridine, bendazac, benoxaprofen, bermoprofen, α-bisabolol, bromfenac, 5-bromosalicylic acid acetate, bromosaligenin, bucloxic acid, butibufen, carprofen, celecoxib, chromoglycate, cinmetacin, clindanac, clopirac, sodium diclofenac, diflunisal, ditazol, droxicam, enfenamic acid, etodolac, etofenamate, felbinac, fenbufen, fenclozic acid, fendosal, fenoprofen, fentiazac, fepradinol, flufenac, flufenamic acid, flunixin, flunoxaprofen, flurbiprofen, glutametacin, glycol salicylate, ibufenac, ibuprofen, ibuproxam, indomethacin, indoprofen, isofezolac, isoxepac, isoxicam, ketoprofen, ketorolac, lornoxicam, loxoprofen, meclofenamic acid, mefenamic acid, meloxicam, mesalamine, metiazinic acid, mofezolac, montelukast, mycophenolic acid, nabumetone, naproxen, niflumic acid, nimesulide, olsalazine, oxaceprol, oxaprozin, oxyphenbutazone, paracetamol, parsalmide, perisoxal, phenyl-acethyl-salicylate, phenylbutazone, phenylsalicylate, pyrazolac, piroxicam, pirprofen, pranoprofen, protizinic acid, reserveratol, salacetamide, salicylamide, salicylamide-O-acetyl acid, salicylsulphuric acid, salicin, salicylamide, salsalate, sulindac, suprofen, suxibutazone, tamoxifen, tenoxicam, theophylline, tiaprofenic acid, tiaramide, ticlopridine, tinoridine, tolfenamic acid, tolmetin, tropesin, xenbucin, ximoprofen, zaltoprofen, zomepirac, tomoxiprol, zafirlukast and cyclosporine. Additional NSAID genera and particular NSAID compounds are disclosed in U.S. Pat. No. 6,297,260 (especially in the generic formulas of its claim 1 and the recitation of specific list of NSAIDs contained therein and in claim 3, and thiazulidene NSAIDs disclosed in International Patent Application WO 01/87890). Preferred NSAIDs are indomethacin, flufenamic acid, flunixin and theophylline. Most preferred is indomethacin, diclofenac and naproxen. In certain embodiments, the NSAID subunit is neither acetyl salicylic acid or mycophenolic acid. Additionally, The Merck Manual, 16th Edition, Merck Research Laboratories (1990) pp 1308-1309, as well as The Pharmacological Basis of Therapeutics, 9th edition, Macmillan Publishing Co., 1996, pp 617-655, provide well known examples of NSAIDs.

The following references describe various NSAIDs suitable for use in the invention described herein, and processes for their manufacture: U.S. Pat. No. 3,558,690 to Sallmann and Pfister, (assigned to Ciba Geigy), issued 1971; U.S. Pat. No. 3,843,681 (assigned to American Home Products), issued 1974; U.S. Pat. No. 3,766,263 to Godfrey, (assigned to Reckitt and Colman) issued 1973; U.S. Pat. No. 3,845,215 to Godfrey (assigned to Reckitt and Colman) issued 1974; U.S. Pat. No. 3,600,437 to Marshall (assigned to EH Lilly), issued 1971; U.S. Pat. No. 3,228,831 to Nicholson and Adams, (assigned to Boots Pure Drug), issued 1966; (U.S. Pat. No. 3,385,886 to Nicholson and Adams, (assigned to Boots Pure Drug) issued 1968; U.S. Pat. No. 3,161,654 to Shen, (assigned to Merck Et Co.), issued 1964; U.S. Pat. No. 3,904,682 to Fried and Harrison, (assigned to Syntex), issued 1975; U.S. Pat. No. 4,009,197 to Fried and Harrison, (assigned to Syntex), issued 1977; U.S. Pat. No. 3,591,584 to Lombardino (assigned to Pfizer) issued 1971; U.S. Pat. No. 5,068,458 to Dales et al., (assigned to Beecham Group, PLC), issued Nov. 26, 1991; U.S. Pat. No. 5,008,283 to Blackburn et al. (assigned to Pfizer, Inc.), issued Apr. 16, 1991; and U.S. Pat. No. 5,006,547 to Loose (assigned to Pfizer), issued Apr. 9, 1991.

Preferred NSAIDs are those agents which have been marketed to the public.

As used herein, the term "prostaglandin mimetic" refers to MNA molecule. Without being bound by theory, it is believed that the stimulation of the release of particular prostaglandins counteract the deleterious effects (or a symptom of the deleterious effects) associated with NSAID use (e.g., GI toxicity).

The language "deleterious effects associated with NSAID use," as used herein, describes diseases and disorders that may be treated or prevented (or a symptom of such disease or disorder that may be reduced) by the prostaglandin mimetic.

Alternatively, the "deleterious effects associated with NSAID use" can describe diseases and disorders that may be treated or prevented (or a symptom of such disease or disorder that may be reduced) by the prostaglandin mimetic used in the composition of the invention in that the prostaglandin mimetic selectively stimulate the release of a prostaglandin. A prostaglandin mimetic that "selectively stimulates the release of a prostaglandin" does not act directly on receptors that bind prostaglandins but creates elevated levels of a particular prostaglandin that are available for the increased binding of that prostaglandin to its target receptor(s). Such prostaglandin mimetics, i.e. MNA, are referred to herein as "selective prostaglandin mimetics."

"NSAID-associated diseases and disorders" refers to any disease, disorder or condition that may be treated in a subject in need thereof with NSAID administration. Examples of NSAID-associated diseases and disorders include, but are not limited to, pain and inflammation.

As used herein, the term "pain" includes all types of pain. Pain includes, but is not limited to, chronic pains, such as arthritis pain (e.g., pain associated with osteoarthritis and rheumatoid arthritis), neuropathic pain, and post-operative pain, chronic lower back pain, cluster headaches, herpes neuralgia, phantom limb pain, central pain, dental pain, neuropathic pain, opioid-resistant pain, visceral pain, surgical pain, bone injury pain, pain during labor and delivery, pain resulting from burns, including sunburn, post partum pain, migraine, angina pain, and genitourinary tract-related pain including cystitis. The term also refers to nociceptive pain or nociception. The term shall refer to persistent pains, such as, but not limited to, neuropathic pain, diabetic neuropathy, fibromyalgia, pain associated with somatoform disorders, arthritic pain, cancer pain, neck pain, shoulder pain, back pain, cluster headaches, tension-type headache, migraine, herpes neuralgia, phantom limb pain, central pain, dental pain, pain traditionally resistant to treatment with NSAIDs, and post-operative pain. The term pain also refers to the pain associated with the inflammation-related diseases and disorders described herein.

The term "inflammation" and "inflammation-related disease and disorder" will be understood by those skilled in the art to include any condition characterized by a localized protective response elicited by injury or destruction of tissues resulting from any of the causes mentioned hereinbefore, and which is manifest by heat, swelling, pain, redness, dilation of blood vessels and/or increased blood flow, invasion of the affected area by white blood cells, loss of function and/or any other symptoms known to be associated with the inflammatory condition. The term will thus be understood to include, inter alia, acute, chronic, ulcerative, specific, allergic and necrotic inflammation, as well as all other forms of inflammation known to those skilled in the art. The term also includes arthritis (including osteoarthritis and rheumatoid arthritis), inflammatory bowel disease, eczema, psoriasis, atopic dermatitis, psoriatic arthropathy and asthma, post-operative inflammation, dental inflammation, acute and chronic ocular inflammatory diseases, conjunctivitis. The compositions of the subject invention advantageously can block the immunogenic inflammatory pathway, thereby providing a method for inhibiting immunogenic inflammation. Accordingly, the subject compositions can be useful in the treatment of neurogenic inflammation, present in different processes, such as diabetes, asthma, cystitis, gingivitis, migraine, dermatitis, rhinitis, psoriasis, inflammation of sciatic and lumbar nerves, gastrointestinal processes, ocular inflammation, and acute allergic response, asthma, rheumatoid arthritis, osteoarthritis and other inflammatory conditions involving acute and/or chronic joint inflammation in a subject, preferably mammalian, more preferably human.

As used herein, the term "gastrointestinal toxicity" (or "GI toxicity") includes, but is not limited to, the gastrointestinal side effects associated with the administration of one of more NSAIDs to a subject, such as, for example, early and late-forming diarrhea and flatulence, nausea, vomiting, anorexia, leukopenia, anemia, neutropenia, asthenia, abdominal cramping, fever, pain, loss of body weight, dehydration, alopecia, dyspnea, insomnia, dizziness, mucositis, xerostomia, and kidney failure, as well as inflammatory bowel disease, Crohn's disease, gastritis, irritable bowel syndrome, ulcerative colitis, constipation, peptic ulcer, partial enteritis, ulcerative colitis, diverticulitis, gastrointestinal haemorrhagia, regional enteritis, diverticulitis, gastrointestinal lesions, gastrointestinal bleeding, coagulation disorders, hypoprothrombinemia, hemophilia or other bleeding problems, kidney disease and the like. Furthermore, the term "gastrointestinal toxicity" refers to any disease, condition, or disorder, or a symptom of a disease, condition, or disorder related to a decrease in one or more prostaglandins (e.g., PGE2 and PGI2) anywhere in the gastrointestinal tract. In a particular embodiment, the term "gastrointestinal toxicity" refers to any disease, condition, or disorder, or a symptom of a disease, condition, or disorder related to a decrease in one or more prostaglandins (e.g., PGE2 and PGI2) in the stomach.

The compositions of the present invention have "pain alleviating properties," "inflammation alleviating properties," as well as "GI toxicity alleviating properties." The term "pain alleviating properties" is generally defined herein to include the expressions "pain-suppressing," "pain-reducing," and "pain-inhibiting" as the invention is applicable to the alleviation of existing pain, as well as the suppression or inhibition of pain which would otherwise ensue from an imminent pain-causing event. Similarly, the term "inflammation alleviating properties" is generally defined herein to include the expressions "inflammation-suppressing," "inflammation-reducing," and "inflammation-inhibiting" as the invention is applicable to the alleviation of existing inflammation, as well as the suppression or inhibition of inflammation which would otherwise ensue from an imminent inflammation-causing event. Furthermore, the term "GI toxicity alleviating properties" is generally defined herein to include the expressions "GI toxicity-suppressing," "GI toxicity-reducing," and "GI toxicity-inhibiting" as the invention is applicable to the alleviation of existing GI toxicity, as well as the suppression or inhibition of GI toxicity which would otherwise ensue from an imminent GI toxicity-causing event (e.g., the administration of an NSAID to a subject).

The term "treatment" or "treating," as used herein, is defined as the application or administration of a pharmaceutical composition of the invention, to a subject who suffers from pain and/or inflammation, a symptom of pain and/or inflammation or a predisposition toward pain and/or inflammation, with the purpose to cure, heal, alleviate, relieve, alter, remedy, ameliorate, improve or affect the pain and/or inflammation, the symptoms of the pain and/or inflammation or the pain and/or inflammation itself. Furthermore, the term refers to administration of a pharmaceutical composition of the invention, to a subject who suffers from GI toxicity, a symptom of GI toxicity or a predisposition toward GI toxicity, with the purpose to cure, counteract, heal, alleviate, relieve, alter, remedy, ameliorate, improve or affect the GI toxicity, the symptoms of the GI toxicity or the GI toxicity itself. In particular embodiments, the GI toxicity to be treated is associated with administration of one or more NSAIDs. Such treatments may be specifically tailored or modified, based on knowledge obtained from the field of pharmacogenomics.

The term "subject" includes living organisms in which pain and/or inflammation can occur, or which are susceptible to pain, inflammation and/or GI toxicity. The term "subject" includes animals (e.g., mammals, e.g., cats, dogs, horses, pigs, cows, goats, sheep, rodents, e.g., mice or rats, rabbits, squirrels, bears, primates (e.g., chimpanzees, monkeys, gorillas, and humans)), as well as chickens, ducks, geese, and transgenic species thereof, and cells derived therefrom. In a preferred embodiment, the term "subject" refers to a human. In one embodiment, the subject is a human with an increased risk for GI toxicity, such as one suffering from diabetes. Administration of the compositions of the present invention to a subject to be treated can be carried out using known procedures, at dosages and for periods of time effective to treat pain and/or inflammation, or GI toxicity, in the subject. In certain embodiments, the GI toxicity in the subject is associated with NSAID use. An effective amount of the therapeutic compositions necessary to achieve a therapeutic effect may vary according to factors such as the state of the disease or disorder in the subject, the age, sex, and weight of the subject, and the ability of the therapeutic compositions to inhibit the pain and/or inflammation in the subject. Dosage regimens can be adjusted to provide the optimum therapeutic response. For example, several divided doses may be administered daily or the dose may be proportionally reduced as indicated by the exigencies of the therapeutic situation. A non-limiting example of an effective dose range for the compound of the invention (e.g., a 1-methylnicotinamide salt in combination with an NSAID) is between 0.25 and 500 mg/kg of body weight/per day. One of ordinary skill in the art would be able to study the relevant factors and make the determination regarding the effective amount of the therapeutic compound without undue experimentation. Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

In particular, the selected dosage level will depend upon a variety of factors including the activity of the particular compound of the present invention employed, the time of administration, the rate of excretion of the particular NSAID being employed, the duration of the treatment, other drugs, compounds or materials used in combination with the particular composition employed, the age, sex, weight, condition, general health and prior medical history of the patient being treated, and like factors well known in the medical arts.

A medical doctor, e.g., physician or veterinarian, having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

The regimen of administration can affect what constitutes an effective amount. The therapeutic formulations can be administered to the subject either prior to or after the onset of pain and/or inflammation or GI toxicity. For example, a 1-methylnicotinamide salt can be administered with an NSAID to counter the GI toxicity associated with NSAID use, or 1-methylnicotinamide can be administered alone, for example, after administration of an NSAID. Further, several divided dosages, as well as staggered dosages, can be administered daily or sequentially. Further, the dosages of the therapeutic formulations can be proportionally increased or decreased as indicated by the exigencies of the therapeutic or prophylactic situation.

In particular embodiments, it is especially advantageous to formulate compositions in dosage unit form for ease of administration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical vehicle. The specification for the dosage unit forms of the invention are dictated by and directly dependent on (a) the unique characteristics of the therapeutic compound and the particular therapeutic effect to be achieved, and (b) the limitations inherent in the art of compounding/formulating such a therapeutic compound for the treatment of a pain, inflammation and/or GI toxicity in subjects.

The nicotinamide derivative of the invention can be synthesized using techniques well-known to the one skilled in the art.

In a preferred embodiment of the invention, the composition comprising the nicotinamide derivative of Formula (I) in combination with one or more NSAIDs, exhibits pain and/or inflammation alleviating properties in subject. In another preferred embodiment of the invention, the nicotinamide derivative of Formula I, alone or in combination with one or more NSAIDs, counteracts GI toxicity associated with administration of NSAIDs. In a preferred embodiment, the subject is a mammal. In another preferred embodiment, the subject is a human.

1-Methylnicotinamide is used as cytoprotective agents (i.e., "gut-sparing").

A particularly preferred pharmaceutical composition of the invention consists of a 1-methylnicotinamide salt and naproxen for use in the treatment of pain and/or inflammation in a subject in need thereof.

Another particularly preferred pharmaceutical composition of the invention consists of a 1-methylnicotinamide salt and indomethacin for use in the treatment of pain and/or inflammation in a subject in need thereof.

Another particularly preferred pharmaceutical composition of the invention consists of a 1-methylnicotinamide salt and diclofenac for use in the treatment of pain and/or inflammation in a subject in need thereof.

The compound of formula (I) can be used to treat gastrointestinal toxicity in a subject.

Another particularly preferred pharmaceutical composition of the invention consists of a 1-methylnicotinamide salt and aspirin for use in the treatment of pain and/or inflammation in a subject in need thereof.

Another particularly preferred pharmaceutical composition of the invention consists of a 1-methylnicotinamide salt and aspirin for use in the treatment of gastrointestinal toxicity in a subject in need thereof.

In another embodiment, the compound of Formula (I) can be administered to a subject to counteract the adverse side-effects, e.g., gastrointestinal toxicity, of an NSAID. In yet another embodiment, the chloride salt of MNA is administered to a subject to counteract the adverse side-effects, e.g., gastrointestinal toxicity, of an NSAID. In still another embodiment NMA is administered to a subject to treat gastrointestinal toxicity

As used herein, the language "pharmaceutically acceptable salt" refers to a salt of the administered compounds prepared from pharmaceutically acceptable non-toxic acids including inorganic acids, organic acids, solvates, hydrates, or clathrates thereof. Examples of such inorganic acids are hydrochloric, hydrobromic, hydroiodic, nitric, sulfuric, and phosphoric. Appropriate organic acids may be selected, for example, from aliphatic, aromatic, carboxylic and sulfonic classes of organic acids, examples of which are formic, acetic, propionic, succinic, camphorsulfonic, citric, fumaric, gluconic, isethionic, lactic, malic, mucic, tartaric, para-toluenesulfonic, glycolic, glucuronic, maleic, furoic, glutamic, benzoic, anthranilic, salicylic, phenylacetic, mandelic, embonic (pamoic), methanesulfonic, ethanesulfonic, pantothenic, benzenes[upsilon]lfonic (besylate), stearic, sulfanilic, alginic, galacturonic, and the like. Preparing a "pharmaceutically acceptable salt" results in the compound of Formula (I) having a "physiologically suitable counter-anion," e.g., Cl⁻.

In accordance with an aspect of the present invention there is disclosed a method of achieving a therapeutic effect for treating a patient suffering from pain and/or and inflammation-related disease or disorder comprising administering a therapeutically effective amount of a pharmaceutical combination comprising as active ingredients (i) a prostaglandin mimetic of Formula (I), or an acceptable salt thereof); and (ii) an NSAID or a pharmaceutically acceptable salt thereof to the patient. In accordance with another aspect of the present invention there is disclosed a method of achieving a therapeutic effect for treating a patient suffering from GI toxicity, as well as an NSAID-associated disease or disorder by administering a therapeutically effective amount of a pharmaceutical combination comprising as active ingredients (i) a prostaglandin mimetic of Formula (I); and (ii) an NSAID or a pharmaceutically acceptable salt thereof to the patient. In accordance with another aspect of the present invention there is provided a method of achieving a therapeutic effect for treating a patient suffering from GI toxicity comprising administering a therapeutically effective amount of a pharmaceutical combination comprising as active ingredients (i) a 1-methylonicotinamide salt; and (ii) an NSAID or a pharmaceutically acceptable salt thereof to the patient.

In another embodiment of this aspect of the present invention the therapeutic effect achieved may be synergistic, in that, the therapeutic effect is greater than the sum of the therapeutic effect achieved by the administration of the active ingredients separately.

In some embodiments, a prostaglandin mimetic of Formula (I) and one or more NSAIDs are included in a single composition, which is administered to a subject experiencing pain and/or inflammation, or GI toxicity. In other embodiments, a nicotinamide derivative of Formula (I) and one or more NSAIDs are administered separately to such a subject. The first and at least one second compound may either be co-administered to a subject (i.e., at the same time) or be administered sequentially (i.e., one after the other).

A combination of compounds described herein can either result in synergistic increase in effectiveness against pain and/or inflammation, relative to effectiveness following administration of each compound when used alone, or such an increase can be additive. Compositions described herein typically include lower dosages of each compound in a composition, thereby avoiding adverse interactions between compounds and/or harmful side effects, such as ones which have been reported for similar compounds. Furthermore, normal amounts of each compound when given in combination could provide for greater efficacy in subjects who are either unresponsive or minimally responsive to each compound when used alone.

A synergistic effect can be calculated, for example, using suitable methods such as, for example, the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 1 14: 313-326 (1926)) and the median- effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

Each of the components of the pharmaceutical composition of the invention ((i) a nicotinamide of Formula (I); and (ii) an NSAID for administration can individually be in the range of from about 1 ng to about 10,000 mg, about 5 ng to about 9,500 mg, about 10 ng to about 9,000 mg, about 20 ng to about 8,500 mg, about 30 ng to about 7,500 mg, about 40 ng to about 7,000 mg, about 50 ng to about 6,500 mg, about 100 ng to about 6,000 mg, about 200 ng to about 5,500 mg, about 300 ng to about 5,000 mg, about 400 ng to about 4,500 mg, about 500 ng to about 4,000 mg, about 1 µg to about 3,500 mg, about 5 µg to about 3,000 mg, about 10 µg to about 2,600 mg, about 20 µg to about 2575 mg, about 30 µg to about 2,550 mg, about 40 µg to about 2,500 mg, about 50 µg to about 2,475 mg, about 100 µg to about 2,450 mg, about 200 µg to about 2425 mg, about 300 µg to about 2,000, about 400 µg to about 1,175 mg, about 500 µg to about 1,150 mg, about 0. 5 mg to about 1,125 mg, about 1 mg to about 1,100 mg, about 1.25 mg to about 1,075 mg, about 1.5 mg to about 1,050 mg, about 2.0 mg to about 1,025 mg, about 2.5 mg to about 1,000 mg, about 3.0 mg to about 975 mg, about 3.5 mg to about 950 mg, about 4.0 mg to about 925 mg, about 4.5 mg to about 900 mg, about 5 mg to about 875 mg, about 10 mg to about 850 mg, about 20 mg to about 825 mg, about 30 mg to about 800 mg, about 40 mg to about 775 mg, about 50 mg to about 750 mg, about 100 mg to about 725 mg, about 200 mg to about 700 mg, about 300 mg to about 675 mg, about 400 mg to about 650 mg, about 500 mg, or about 525 mg to about 625 mg.

In some embodiments, dose of a nicotinamide derivative of the invention for treatment of pain and/or inflammation or GI toxicity is between about 0.0001 mg and about 25 mg In some embodiments, a dose of a nicotinamide derivative of the invention used in compositions described herein is less than about 100 mg, or less than about 80 mg, or less than about 60 mg, or less than about 50 mg, or less than about 30 mg, or less than about 20 mg, or less than about 10 mg, or less than about 5 mg, or less than about 2 mg, or less than about 0.5 mg. Similarly, in some embodiments, a dose of a second compound (i.e., an NSAID) as described herein is less than about 1000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg.

In one embodiment, the prostaglandin mimetic of Formula (I) (MNA) is administered first followed by administration of an NSAID. In another embodiment, the NSAID is administered first followed by administration of the prostaglandin mimetic of Formula (I) (MNA). In still another embodiment, the prostaglandin mimetic of Formula (I) (MNA) and NSAID are administered at the same time.

### Formulations for Administration

In another embodiment, the present invention is directed to a packaged pharmaceutical composition comprising a container holding a therapeutically effective amount of a nicotinamide of Formula (I), alone and in combination with one or more NSAIDs; and instructions for using the compound to treat, prevent, or reduce one or more symptoms of one or more pain, inflammation and/or GI toxicity related conditions in a subject.

The term "container" includes any receptacle for holding the pharmaceutical composition. For example, in one embodiment, the container is the packaging that contains the pharmaceutical composition. In other embodiments, the container is not the packaging that contains the pharmaceutical composition, i.e., the container is a receptacle, such as a box or vial that contains the packaged pharmaceutical composition or unpackaged pharmaceutical composition and the instructions for use of the pharmaceutical composition. Moreover, packaging techniques are well known in the art. It should be understood that the instructions for use of the pharmaceutical composition may be contained on the packaging containing the pharmaceutical composition, and as such the instructions form an increased functional relationship to the packaged product. However, it should be understood that the instructions can contain information pertaining to the compound's ability to perform its intended function, e.g., treating, preventing, or reducing one or more pain, inflammation and/or GI toxicity-related abnormalities in a subject.

Another embodiment of the invention is a pharmaceutical composition comprising a therapeutically effective amount of a nicotinamide compound of Formula I and a pharmaceutically acceptable carrier. The language "therapeutically effective amount" describes the amount of nicotinamide derivative of Formula I of the invention that is effective to treat one or more symptoms of pain and/or inflammation in a subject.

The language "pharmaceutically acceptable carrier" includes a pharmaceutically acceptable material, composition or carrier, such as a liquid or solid filler, diluent, excipient, solvent or encapsulating material, involved in carrying or transporting a compound(s) of the present invention within or to the subject such that it can perform its intended function. Typically, such compounds are carried or transported from one organ, or portion of the body, to another organ, or portion of the body. Each carrier must be "acceptable" in the sense of being compatible with the other ingredients of the formulation, and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethylcellulose, ethyl cellulose and cellulose acetate; powdered tragacanth; malt; gelatin; talc; and other non-toxic compatible substances employed in pharmaceutical formulations. As used herein "pharmaceutically acceptable carrier" also includes any and all coatings, antibacterial and antifungal agents, and absorption delaying agents, and the like that are compatible with the activity of the compound, and are physiologically acceptable to the subject. Supplementary active compounds can also be incorporated into the compositions.

The compounds for use in the invention are formulated for administration by oral route.

Suitable compositions and dosage forms include, for example, tablets, capsules, caplets, pills, gel caps, troches, dispersions, suspensions, solutions, syrups, granules, beads, transdermal patches, gels, powders, pellets, magmas, and lozenges. It should be understood that the formulations and compositions that would be useful in the present invention are not limited to the particular formulations and compositions that are described herein.

For oral administration the compounds can be in the form of tablets or capsules prepared by conventional means with pharmaceutically acceptable excipients such as binding agents (e.g., polyvinylpyrrolidone, hydroxypropylcellulose or hydroxypropylmethylcellulose); fillers (e.g., cornstarch, lactose, microcrystalline cellulose or calcium phosphate); lubricants (e.g., magnesium stearate, talc, or silica); disintegrants (e.g., sodium starch glycollate); or wetting agents (e.g., sodium lauryl sulphate). If desired, the tablets can be coated using suitable methods and coating materials such as OP ADR Y(TM) film coating systems available from Colorcon, West Point, Pa. (e.g., OPADRY(TM) OY Type, OY-C Type, Organic Enteric OY-P Type, Aqueous Enteric OY-A Type, OY-PM Type and OPADRY(TM) White, 32Kl 8400). Liquid preparation for oral administration can be in the form of solutions, syrups or suspensions. The liquid preparations can be prepared by conventional means with pharmaceutically acceptable additives such as suspending agents (e.g., sorbitol syrup, methyl cellulose or hydrogenated edible fats); emulsifying agent (e.g., lecithin or acacia); non-aqueous vehicles (e.g., almond oil, oily esters or ethyl alcohol); and preservatives (e.g., methyl or propyl p-hydroxy benzoates or sorbic acid).

### Controlled Release Formulations and Drug Delivery Systems

In certain embodiments, the formulations of the present invention can be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time can be as long as a month or more and should be a release which is longer that the same amount of agent administered in bolus form. For sustained release, the compounds can be formulated with a suitable polymer or hydrophobic material which provides sustained release properties to the compounds.

In a preferred embodiment of the invention, the nicotinamide compounds of Formula (I) are administered to a subject, alone or in combination with an NSAID, using a sustained release formulation.

The term delayed release is used herein in its conventional sense to refer to a drug formulation that provides for an initial release of the drug after some delay following drug administration which, although not necessarily, includes a delay of from about 10 minutes up to about 12 hours. The term pulsatile release is used herein in its conventional sense to refer to a drug formulation that provides release of the drug in such a way as to produce pulsed plasma profiles of the drug after drug administration.

The term immediate release is used in its conventional sense to refer to a drug formulation that provides for release of the drug immediately after drug administration. As used herein, short-term refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes after drug administration.

As used herein, rapid-offset refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes after drug administration.

### Dosing

The therapeutically effective amount or dose of a compound of the present invention will depend on the age, sex and weight of the patient, the current medical condition of the patient and the nature of the pain, inflammation and/or GI-toxicity being treated. The skilled artisan will be able to determine appropriate dosages depending on these and other factors. A suitable dose of a compound of the present invention can be in the range of from about 0.001 mg to about 1000 mg per day, such as 0.001 mg to about 500 mg per day, such as from about 0.01 mg to about 100 mg, for example, from about 0.05 mg to about 50 mg, such as about 0.5 mg to about 25 mg per day. The dose can be administered in a single dosage or in multiple dosages, for example from 1 to 4 or more times per day. When multiple dosages are used, the amount of each dosage can be the same or different. For example a dose of 1 mg per day can be administered as two 0.5 mg doses, with about a 12 hour interval between doses.

It is understood that the amount of compound dosed per day can be administered every day, every other day, every 2 days, every 3 days, every 4 days, every 5 days, etc. For example, with every other day administration, a 5 mg per day dose can be initiated on Monday with a first subsequent 5 mg per day dose administered on Wednesday, a second subsequent 5 mg per day dose administered on Friday, etc.

The compounds for use in the method of the invention can be formulated in unit dosage form. The term "unit dosage form" refers to physically discrete units suitable as unitary dosage for subjects undergoing treatment, with each unit containing a predetermined quantity of active material calculated to produce the desired therapeutic effect, optionally in association with a suitable pharmaceutical carrier. The unit dosage form can be for a single daily dose or one of multiple daily doses (e.g., about 1 to 4 or more times per day). When multiple daily doses are used, the unit dosage form can be the same or different for each dose.

### Exemplification of the Invention

The invention is further illustrated by the following example, which could be used to examine prostaglandin-releasing activity of the compounds of the invention. The example should not be construed as further limiting. The animal models used throughout the Examples are accepted animal models and the demonstration of efficacy in these animal models is predictive of efficacy in humans.

### Example 1:

Thrombolytic activity of the prostaglandin mimetic of the invention can be assessed using the assay described in WO 2005/067927. This assay was used to demonstrate that 1-methylnicotinamide chloride has the ability to induce the release of prostacyclin in animal models.

### Example 2:

In this study, streptozocin (70 mg/kg injected intraperitoneally) was used to induce diabetes mellitus and non-diabetic and diabetic rats were pretreated 30 min prior to exposure to aspirin (ASA; 150 mg/kg in 0.1 N HCl i.g.) or 3.5 h of water immersion and restraint stress (WRS) with 1) vehicle (saline) or 2) MNA (2.5 - 50 mg/kg i.g.). The area and number of gastric lesions was determined by planimetry, gastric blood flow (GBF) was examined by H2-gas clearance technique, and mieloperoxidase (MPO) activity, activity of superoxide dismutase (SOD) and the malonyldialdehyde (MDA) concentration as an index of lipid peroxidation were determined in gastric mucosa using ELISA. The gene expression IL-1β and TNF-α in the gastric mucosa and their plasma levels were evaluated by RT-PCR and ELISA, respectively. ASA and WRS caused typical gastric lesions and reduced significantly GBF (by 35% and 28% from basal) and increased MPO activity (2-3 fold) and gastric mucosal MDA content and these effects were significantly augmented in diabetic rats (plasma glucose < 400 mg/dL). The ulcerogenic effects of ASA or WRS were accompanied by the fall in GBF and the overexpression of mRNAs for TNF-α and IL-1β, as well as a marked increase in their plasma levels. MNA dose-dependently attenuated ASA- and WRS-induced gastric erosions in diabetic and non-diabetic animals while raising GBF in intact and injured gastric mucosa by about 22% and 33%, respectively, and caused downregulation of IL-1β and TNF-α. The mucosal SOD activity was significantly decreased while the MDA activity was significantly increased in ASA- and WRS-exposed animals without or with diabetes and these effects were markedly attenuated by MNA. These studies demonstrate that 1) diabetes enhances the susceptibility of gastric mucosa to the damage induced by ASA and WRS via mechanism involving fall in GBF, suppression of SOD activity, an increase in lipid peroxidation and overexpression of proinflammatory cytokines IL-β and TNF-α, and 2) MNA affords protection against ASA- and WRS- induced damage. This experiment is also described in an abstract published for Digestive Disease Week 2006 (May 20-25, 2006, Los Angeles, California; http://www.medscape.com/viewprogram/5452).

## Claims

1. A pharmaceutical composition comprising a Nonsteroidal Anti-inflammatory Drug (NSAID) and a 1-methylnicotinamide salt of formula (I): 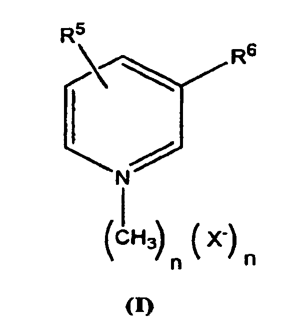 wherein R⁵ is H; R⁶ is -CONH₂; n is 1; and X⁻ is a physiologically suitable counter-anion; wherein the composition is formulated for oral administration.

2. The composition of claim 1, wherein X⁻ is chloride, benzoate, salicylate, acetate, citrate or lactate anion.

3. The composition of claim 1, wherein the compound of formula (I) is selected from 1-methylnicotinamide chloride, 1-methylnicotinamide citrate, and 1-methylnicotinamide lactate.

4. The composition of any one of claims 1 to 3, wherein the NSAID is selected from the group consisting of indomethacin, ibuprofen, naproxen, fenoprofen, tolmetin, sulindac, meclofenamate, ketoprofen, piroxicam, flurbiprofen, diclofenac, acetylsalicylic acid, sodium acetylsalicylate, calcium acetylsalicylate, sodium salicylate, choline salicylate, magnesium salicylate, salsalate, diflunisal, ketorolac, carprofen, mefenamic acid, meloxicam and nimesulide.

5. The composition of any one of claims 1 to 4, the composition being in a form of a tablet, a capsule, solution, syrup or suspension.

6. The pharmaceutical composition as defined in any one of claims 1 to 5 for use in a method of treating pain and/or inflammation in a subject.

7. The pharmaceutical composition of any one of claims 1 to 5 for use according to claim 6, wherein the inflammation is selected from the group consisting of fever, arthritis, asthma, bronchitis, menstrual cramps, tendinitis, bursitis, inflammatory disorders of the skin, gastrointestinal conditions, vascular diseases, migraine headaches, periarteritis nodosa, thyroiditis, aplastic anemia, Hodgkin's disease, sclerodoma, rheumatic fever, myasthenia gravis, sarcoidosis, nephrotic syndrome, Behcet's syndrome, polymyositis, hypersensitivity, conjunctivitis, gingivitis, swelling occurring after injury and myocardial ischemia.

8. The pharmaceutical composition for use according to claim 7, wherein the inflammatory disorders of the skin are selected from the group consisting of psoriasis, eczema, burns and dermatitis.

9. The pharmaceutical composition for use according to claim 6, wherein the pain is selected from the group consisting of menstrual pain, low back pain, neck pain, skeletal pain, post-partum pain, headache, pain associated with migraine, toothache, sprains, strains, arthritis, degenerative joint diseases, gout, ankylosing spondylitis, bursitis, burns, including radiation and corrosive chemical injuries, sunburns, bone fracture, immune and autoimmune diseases, cellular neoplastic transformations or metastic tumor growth, and pain following surgical and dental procedures.

10. The pharmaceutical composition for use according to claim 9, wherein the arthritis is selected from rheumatoid arthritis, spondyloarthopathies, gouty arthritis, systemic lupus erythematosus, osteoarthritis and juvenile arthritis.

## Patentansprüche

1. Pharmazeutische Zusammensetzung, umfassend einen nichtsteroidalen Entzündungshemmer (NSAID) und ein 1-Methylnicotinamidsalz der Formel (I): 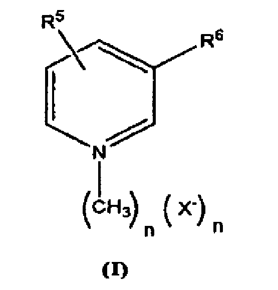 worin R⁵ für H steht; R⁶ für -CONH₂ steht; n 1 ist; und X⁻ ein physiologisch geeignetes Gegenanion ist, wobei die Zusammensetzung für die orale Verabreichung formuliert ist.

2. Zusammensetzung nach Anspruch 1, wobei X⁻ ein Chlorid-, Benzoat-, Salicylat-, Acetat-, Citrat- oder Lactat-Anion ist.

3. Zusammensetzung nach Anspruch 1, wobei die Verbindung der Formel (I) aus 1-Methylnicotinamid-chlorid, 1-Methylnicotinamid-citrat und 1-Methylnicotinamid-lactat ausgewählt ist.

4. Zusammensetzung nach einem der Ansprüche 1 bis 3, wobei der NSAID aus der aus Indomethacin, Ibuprofen, Naproxen, Fenoprofen, Tolmetin, Sulindac, Meclofenamat, Ketoprofen, Piroxicam, Flurbiprofen, Diclofenac, Acetylsalicylsäure, Natriumacetylsalicylat, Calciumacetylsalicylat, Natriumsalicylat, Cholinsalicylat, Magnesiumsalicylat, Salsalat, Diflunisal, Ketorolac, Carprofen, Mefenaminsäure, Meloxicam und Nimesulid bestehenden Gruppe ausgewählt ist.

5. Zusammensetzung nach einem der Ansprüche 1 bis 4, wobei die Zusammensetzung in Form einer Tablette, einer Kapsel, einer Lösung, eines Sirups oder einer Suspension vorliegt.

6. Pharmazeutische Zusammensetzung, wie in einem der Ansprüche 1 bis 5 definiert ist, zur Verwendung in einem Verfahren zur Behandlung von Schmerz und/oder Entzündung bei einem Patienten.

7. Pharmazeutische Zusammensetzung nach einem der Ansprüche 1 bis 5 zur Verwendung gemäß Anspruch 6, wobei die Entzündung aus der aus Fieber, Arthritis, Asthma, Bronchitis, Menstruationsbeschwerden, Tendinitis, Bursitis, entzündlichen Hauterkrankungen, Magen-Darm-Erkrankungen, Gefäßerkrankungen, Migränekopfschmerz, Periarteriitis nodosa, Thyroiditis, aplastischer Anämie, Morbus Hodgkin, Sklerodomie, rheumatischem Fieber, Myasthenia gravis, Sarkoidose, nephrotischem Syndrom, Behçet-Syndrom, Polymyositis, Hypersensitivität, Konjunktivitis, Gingivitis, nach einer Verletzung auftretenden Schwellungen und myokardialer Ischämie bestehenden Gruppe ausgewählt ist.

8. Pharmazeutische Zusammensetzung zur Verwendung gemäß Anspruch 7, wobei die entzündlichen Hauterkrankungen aus der aus Psoriasis, Ekzem, Verbrennungen und Dermatitis bestehenden Gruppe ausgewählt sind.

9. Pharmazeutische Zusammensetzung zur Verwendung gemäß Anspruch 6, wobei der Schmerz aus der aus Menstruationsschmerzen, Kreuzschmerzen, Nackenschmerzen, Skelettschmerzen, Schmerzen nach einer Geburt, Kopfschmerzen, Schmerzen im Zusammenhang mit Migräne, Zahnschmerzen, Zerrungen, Verstauchungen, Arthritis, degenerativen Gelenkserkrankungen, Gicht, ankylosierender Spondylitis, Bursitis, Verbrennungen, einschließlich Verletzungen durch Strahlung und korrosive Chemikalien, Sonnenbrand, Knochenbruch, Immun- und Autoimmunerkrankungen, zellulären neoplastischen Transformationen oder Metastasenwachstum und Schmerzen nach einem operativen oder zahnmedizinischen Eingriff bestehenden Gruppe ausgewählt ist.

10. Pharmazeutische Zusammensetzung zur Verwendung gemäß Anspruch 9, wobei die Arthritis aus rheumatoider Arthritis, Spondyloarthropathien, Gichtarthritis, systemischem Lupus erythematodes, Osteoarthritis und juveniler Arthritis ausgewählt ist.

## Revendications

1. Une composition pharmaceutique comprenant un médicament anti-inflammatoire non stéroïdien (AINS) et un sel de 1-méthylnicotinamide de la formule (I): 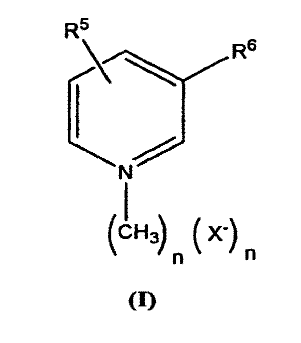 dans laquelle R⁵ représente H; R⁶ représente CONH₂; n est 1; et X⁻ est un contre-ion physiologiquement acceptable; ladite composition étant formulée pour l'administration orale.

2. La composition selon la revendication 1, dans laquelle X⁻ est l'anion chlorure, benzoate, salicylate, acétate, citrate ou lactate.

3. La composition selon la revendication 1, dans laquelle le compose de la formule (I) est choisi parmi le chlorure de 1-méthylnicotinamide, le citrate de 1-méthylnicotinamide et le lactate de 1-méthylnicotinamide.

4. La composition selon l'une quelconque des revendications 1 à 3, dans laquelle l'AINS est choisi parmi le group composé de indométhacine, ibuprofène, naproxène, fénoprofène, tolmetin, sulindac, meclofenamate, kétoprofène, piroxicam, flurbiprofène, diclofénac, acide acétylsalicylique, acétylsalicylate de sodium, acétylsalicylate de calcium, salicylate de sodium, salicylate de choline, salicylate de magnésium, salsalate, diflunisal, kétorolac, carprofène, acide méfénamique, méloxicam et nimésulide.

5. La composition selon l'une quelconque des revendications 1 à 4, dans laquelle la composition est sous forme de comprimé, de capsule, de solution, de sirop ou de suspension.

6. La composition pharmaceutique selon l'une quelconque des revendications 1 à 5 pour utilisation dans la méthode de traitement de la douleur et/ou d'une inflammation chez un sujet.

7. La composition pharmaceutique selon l'une quelconque des revendications 1 à 5 pour utilisation selon la revendication 6, dans laquelle l'inflammation est choisie parmi le group composé de la fièvre, l'arthrite, l'asthme, la bronchite, les crampes menstruelles, la tendinite, la bursite, les troubles inflammatoires de la peau, les troubles gastro-intestinaux, les maladies vasculaires, les migraines, la périartérite noueuse, thyroiditis, l'anémie aplastique, la maladie de Hodgkin, la sclérodermie, le rhumatisme articulaire aigu, la myasthénie grave, la sarcoïdose, le syndrome néphrotique, le syndrome de Behcet, la polymyosite, l'hypersensibilité, la conjonctivite, la gingivite, l'enflure survenant après une blessure et l'ischémie du myocardique.

8. La composition pharmaceutique pour utilisation selon la revendication 7, dans laquelle les troubles inflammatoires de la peau sont choisis parmi le group consistant de le psoriasis, l'eczéma, les brûlures et la dermatite.

9. La composition pharmaceutique pour utilisation selon la revendication 6, dans laquelle la douleur est choisie parmi le group composé de les douleurs menstruelles, les douleurs au bas du dos, la douleur au cou, la douleur squelettique, la douleur post-partum, des maux de tête, douleur associée à la migraine, les maux de dents, les entorses, les foulures, la polyarthrite, les maladies dégénératives des articulations, la goutte, la spondylarthrite ankylosante, la bursite, les brûlures, y compris les lésions induites par radiation et chimiques corrosifs, les coups de soleil, les fractures osseuses, maladies auto-immunes et auto-immunes, des transformations néoplasiques cellulaires ou métastatique croissance de la tumeur, et la douleur après une intervention chirurgicale et dentaire.

10. La composition pharmaceutique pour utilisation selon la revendication 9, dans laquelle la polyarthrite est choisie parmi la polyarthrite rhumatoïde, les spondyloarthropathies, l'arthrite goutteuse, le lupus érythémateux disséminé, l'ostéoarthrite et l'arthrite juvénile.
